# EUROPEAN PATENT APPLICATION

(11) **EP 3 974 017 A1**
(43) Date of publication of application: **30.03.2022**
(21) Application number: 20198798.9
(22) Date of filing: 28.09.2020
(51) Int. Cl.: A61M 37/00, A61M 25/02

(54) **A MICRONEEDLE PATCH APPLICATION SYSTEM**

(71) Applicant: Latch Medical, 4 Dublin (IE)
(72) Inventor: BERTOLLO, Nicky, Dublin, 24 (IE)
(74) Representative: FRKelly

(57) **Abstract**

The present invention provides a microneedle patch application system designed to facilitate the simple and expeditious deployment of a microneedle patch onto a tissue substrate such as the skin, for drug delivery or other applications, the system including a microneedle patch including arrays of opposing microneedles and an applicator operable to deploy the patch onto the tissue substrate by displacing the patch from an undeployed to a deployed state.

## Description

### Field of the invention

The present invention is concerned with a microneedle patch application system designed to facilitate the simple and expeditious deployment of a microneedle patch onto a tissue substrate such as the skin, which microneedle patch may have various therapeutic, surgical, cosmetic or other uses, a primary use being drug delivery, but other uses including bio-sensing and transcutaneous electrical nerve stimulation (TENS) applications, as well as measuring other bioelectrical activity in muscle tissue such as EMG and ECG.

In addition the patch may be used to anchor therapeutic and diagnostic surgical and interventional devices to tissue such as the skin. These include surgical catheters, drains, cannulae and stoma dressings.

### Background of the invention

Across a wide range of surgical and medical procedures it is generally desirable to minimise tissue trauma, which is beneficial in both reducing surgical times and patient recovery, in addition to reducing the risk of infection, minimising the surgical equipment needed and thus potentially the number of surgeons and/or support personal required to perform a given surgical or medical procedure.

International applications WO2018/069543 and WO2019/201903 provide detailed disclosures of the configuration and operation of microneedles and opposing microneedle arrays which may take the form of a patch for application to a tissue substrate for various surgical and therapeutic uses, one particular use being drug delivery from or through the microneedles provided. The disclosures of WO2018/069543 and WO2019/201903 are incorporated herein in their entirety.

In such drug delivery applications, in particular large scale programs such as the administration of a vaccine or the like, it would be highly beneficial to simplify the deployment of such a microneedle patch to enable deployment by an end user, by reducing the complexity, time and effort required for deployment. In this way such a patch could be made directly available to the end user through suitable channels, and the patch could then be deployed onto the skin to deliver the vaccine or other drug without requiring the intervention of a medical professional.

It is therefore an object of the present invention to provide a microneedle patch application system which is operable to quickly and easily deploy a microneedle patch onto a tissue substrate such as the skin, in particular to allow end user deployment without the intervention or supervision of a medical professional.

### Summary of the invention

According to a first aspect of the present invention there is provided a microneedle patch application system comprising a microneedle patch for deployment on a tissue substrate and an applicator operable to deploy the microneedle patch onto said substrate; wherein the microneedle patch comprises two sets of microneedle arrays with the microneedles in one array pointed in generally the opposite direction to the microneedles in the other array, the arrays being displaceable relative to one another between an undeployed state and a deployed state; a coupling operable to releasably retain the microneedle patch on the applicator; and an actuator operable to affect displacement of the microneedle arrays from the undeployed to the deployed state when the patch is retained on the applicator.

Preferably, the applicator is deformable and said deformation affects operation of the actuator and/or the coupling.

Preferably, the applicator comprises a first portion and a second portion and deformation of the applicator is affected by relative displacement between the first and second portions.

Preferably, the system comprises a lock operable to prevent relative displacement of the first and second portions until a threshold pressure is applied to the applicator from the patch.

Preferably, the system comprises a non return lock operable to prevent the first and second portions from undergoing relative displacement once the arrays have been displaced into the deployed state.

Preferably, the applicator is manually deformable.

Preferably, the applicator is operable such that actuation of the actuator affects actuation of the coupling.

Preferably, the coupling and the actuator are integrated with one another such as to facilitate unitary operation thereof.

Preferably, the actuator comprises a pair of first surfaces on the applicator which are displaceable relative to one another, and a pair of second surfaces each of which is secured to one of the arrays, the second surfaces being engageable with and displaceable by the first surfaces.

Preferably, the pair of first surfaces are displaceable relative to one another by means of a linear translation and/or a rotational translation of the pair of first surfaces.

Preferably, the pair of first surfaces are substantially parallel and displaceable relative to one another in a direction oblique to the first surfaces, the pair of second surfaces are substantially parallel and each of which is secured to one of the arrays, the second surfaces being displaceable relative to one another in a direction oblique to the second surfaces, the first and second surfaces being in face to face engagement when the patch is retained on the applicator.

Preferably, the coupling comprises at least one key on the applicator or patch and a corresponding keyway on the other of the applicator or patch within and along which the at least one key is slidably and releasably captured.

Preferably, the keyway is at least partially defined by one of the second surfaces.

Preferably, the key is releasable from the keyway following a predetermined relative displacement of the first surfaces relative to the second surfaces.

According to a second aspect of the present invention there is provided a method of applying a microneedle patch to a tissue substrate comprising the steps of releasably securing a microneedle patch to an applicator, the patch comprising two sets of microneedle arrays with the microneedles in one array pointed in generally the opposite direction to the microneedles in the other array; pressing the arrays of microneedles against the tissue substrate; utilising the applicator to displace the pair of arrays relative to one another between an undeployed state and a deployed state such as to at least partially embed the microneedles in the tissue substrate; and releasing the patch from the actuator.

As used herein, the term "microneedle" is intended to mean a needle which is of a particular dimension, generally in the range of 100-3,000 micrometres (µm) in length or height, and which can be used as a barb and/or drug delivery or bio-sensing system, and generally having a free end or tip for piercing tissue to facilitate at least partial insertion of the microneedle into the tissue, which microneedle can therefore be considered as pointing in the direction in which the tip is facing.

As used herein, the term "opposing" is intended to mean that components in one set or array are pointed generally in one direction and components in an other set or array are pointed in substantially the opposite direction, to include configurations where the components are pointed obliquely in opposed directions, such that the opposed arrays may be displaced relative to one another along a substrate such as tissue in order to engage, grip, pierce and become at least partially inserted or embedded in the substrate along which the opposed arrays are being displaced.

### Brief description of the drawings

The present invention will now be described with reference to the accompanying drawings, in which:
Figure 1 illustrates a perspective view of a microneedle patch application system according to an embodiment of the present invention, located on a tissue substrate and with a microneedle patch of the system in an undeployed state;
Figure 2 illustrates a perspective view, from below, of the microneedle patch application system of Figure 1;
Figure 3 illustrates the microneedle patch application system following actuation of an applicator of the system to displace the patch into a deployed state;
Figure 4 illustrates the microneedle patch application system with the patch secured to the tissue substrate and the applicator being withdrawn;
Figure 5 illustrates an upper face of the microneedle patch when in the undeployed state;
Figure 6 illustrates the upper face of the microneedle patch when in the deployed state;
Figure 7 illustrates an underside of the applicator when in a first configuration corresponding to the undeployed state of the patch;
Figure 8 illustrates the underside of the applicator when in a second configuration corresponding to the deployed state of the patch;
Figure 9 illustrates the sequential steps in operating the microneedle patch application system to deploy the microneedle patch onto the tissue substrate;
Figure 10 illustrates a perspective view from above of the microneedle patch deployed with the tissue substrate;
Figure 11 illustrates the microneedle patch on the tissue substrate but having been manually displaced from the deployed to the undeployed state;
Figure 12 illustrates the microneedle patch being removed from the tissue substrate;
Figure 13 illustrates the microneedle patch having been fully removed from the tissue substrate and remaining in the undeployed state;
Figure 14 illustrates a plan view from above of an alternative embodiment of a microneedle patch forming part of the present invention, and in an undeployed state;
Figure 15 illustrates the microneedle patch of Figure 14 in a deployed state;
Figure 16 illustrates a plan view from above of a further alternative embodiment of a microneedle patch forming part of the present invention, in an undeployed state;
Figure 17 illustrates the microneedle patch of Figure 16 in a deployed state;
Figure 18 illustrates the microneedle patch of Figures 16 and 17 in a removal configuration;
Figure 19 illustrates an actuator for affecting deployment of the patch illustrated in Figures 16 to 18;
Figure 20 illustrates a plan view from above of an embodiment of a microneedle patch forming part of the present invention, in an undeployed state;
Figure 21 illustrates a plan view from above of the microneedle patch of Figure 20 in a deployed state;
Figure 22 illustrates a perspective view of the patch shown in Figures 20 and 21;
Figure 23 illustrates a perspective view from above of an additional embodiment of a microneedle patch forming part of the present invention, in an undeployed state and partially illustrating an applicator engaged therewith;
Figure 24 illustrates a plan view of the arrangement of Figure 23, with the patch having been displaced into a deployed state;
Figure 25 illustrates a plan view of the patch shown in Figure 23 in isolation from the applicator, in the deployed state and with protrusions depressed in preparation for removal; and
Figure 26 a plan view of the patch as shown in Figure 25 following removal from skin or other tissue substrate.

### Detailed description of the drawings

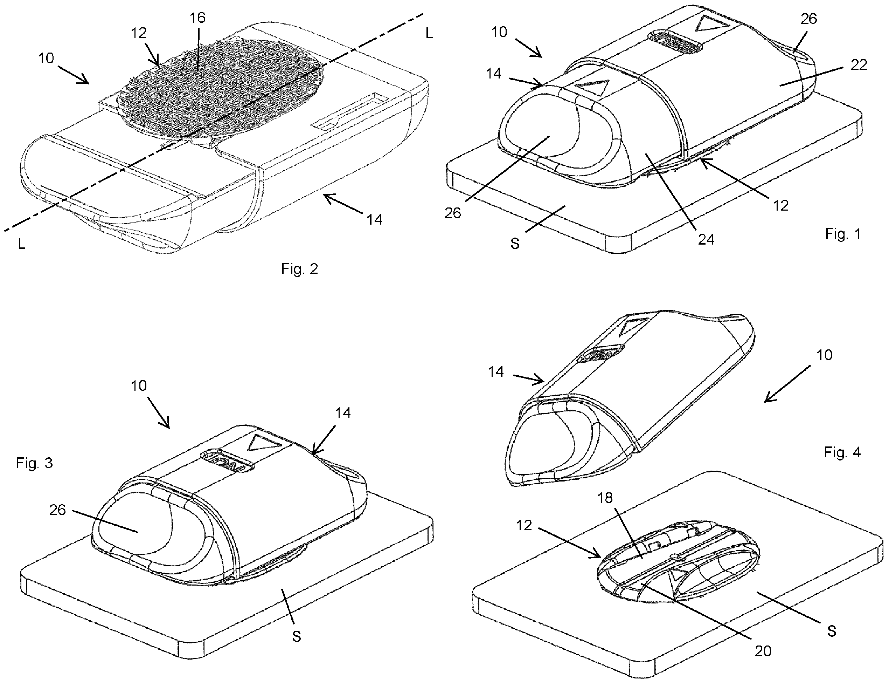

Referring now to Figures 1 to 13 there is illustrated a microneedle patch application system according to an embodiment of the present invention, and generally indicated as 10. The system 10 comprises as the primary components a microneedle patch 12 as described in detail hereinafter, and an applicator 14 adapted to facilitate the fast and effective deployment of the patch 12 onto a tissue substrate such as skin S. The microneedle patch 12 may provide various therapeutic and/or surgical functions, and for example may be used for transdermal drug delivery. There may also be non medical applications for the patch 12, such as for tattoo removal or as a mechanical mounting or fastening.

The patch 12 comprises two arrays of microneedles 16, the microneedles 16 in one array being pointed in generally the opposite direction to the microneedles 16 in the other array, the two arrays being displaceable relative to one another to displace the microneedles 16 between an undeployed state and a deployed state. The patch 12 comprises first and second patch halves 18, 20 which are slidably engaged with one another, each patch half 18, 20 carrying one of the arrays of microneedles 16 on a skin facing surface thereof. The patch halves 18, 20 may be formed from any suitable material or combination of materials, and are preferably formed from moulded plastic. The microneedles 16 may be formed from a different material, for example metal, and may be secured to the patch halves 18, 20 by any suitable means. The patch halves 18, 20 may also include instructional iconography and/or colour coding or the like in order to assist the user, for example to effect removal of the patch 12 and as will be described hereinafter.

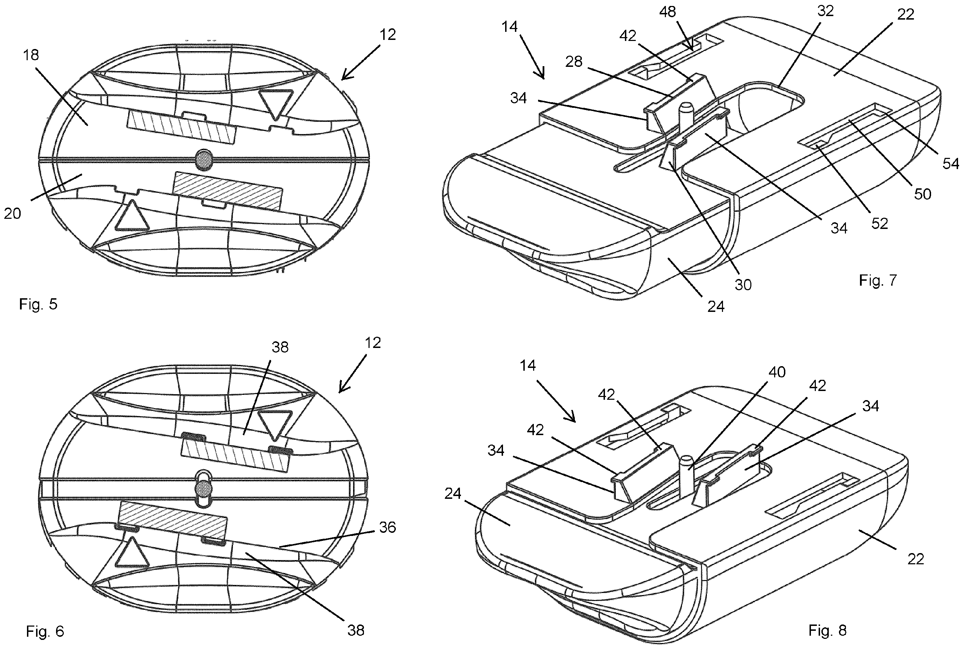

The patch halves 18, 20 are displaceable relative to one another, and in use transversely of a longitudinal axis LL of the system 10, by a fixed distance in order to displace the arrays of microneedles 16 between the undeployed and deployed states, as shown for example in Figures 5 and 6. The arrays of microneedles 16 may be provided in a large number of configurations, and for example may be arranged with the microneedles 16 of each array configured in parallel spaced rows, with the rows of the two arrays being interlaced with one another. This configuration, along with various other configurations, physical and operational attributes of the microneedles 16, are disclosed in detail in earlier International applications WO2018/069543 and WO2019/201903, and thus no further detail regarding the microneedles is required in the present application.

The applicator 14 is adapted to retain the patch 12 against the underside of the applicator 14 with the patch 12 in the undeployed state, and while the microneedles 16 are being pressed against the skin S or other tissue substrate, to sequentially displace the patch 12 into the deployed state and release the patch 12 from the applicator 14, so leaving the patch 12 deployed on the skin S to perform the intended function, for example transdermal drug delivery via the microneedles 16. The applicator 14 is thus adapted to convert manual user input as applied via the applicator 14 into displacement of the patch 12 from the undeployed to the deployed state. In the embodiment illustrated the undeployed state is defined by the patch halves 18, 20 being fully retracted relative to one another as shown in Figure 5, and so may be referred to as the patch 12 being "closed". Conversely the deployed stage of the patch is defined by the patch halves 18, 20 being extended relative to one another as shown in Figure 6, and so may be referred to as the patch 12 being "open". A longitudinally extending channel or gap can be observed in Figure 6 between the patch halves 18, 20 with the patch open and which is indicative of the distance that the patch halves 18, 20 are displaced relative to one another between the undeployed and deployed states. It will of course be understood that the dimensions and geometry of the patch 12 may be significantly varied while still retaining the functionality described herein.

The applicator comprises a first body portion 22 and a second body portion 24 slidably displaceable relative to one another in a direction along the longitudinal axis LL of the system 10 and by a fixed distance. In the embodiment illustrated the second body portion 24 is telescopically received within the first body portion 22 but any other suitable mechanical configuration may be employed. In this way the applicator 14 can be deformed via relative displacement of the body portions 22, 24 and this deformation can be arranged to affect displacement of the patch 12 between the closed and open or undeployed and deployed stages, and or to affect release of the patch 12 once in the deployed state, as will be described in detail hereinafter. The free or outer end of each body portion 22, 24 is preferably provided with a recess 26 shaped and dimensioned to receive the tip of an actuating digit, most preferably the index finger and thumb of the user, allowing the user to securely grip the applicator 14 during deformation of the applicator 14 and thus deployment of the patch 12. The recesses 26 also serves to ensure that the finger and thumb of the user do not extend below the underside of the applicator 14 such as to contact the skin S during deployment, which could negatively impact the extent of the downward pressure to be applied at the tips of the microneedles 16 to ensure insertion into the skin S. Referring to Figure 4 it can also be see that the underside of the body portions 22, 24 directly beneath the respective recess 26 is contoured, curving downwardly away from the free end of the body portion 22, 24 towards the patch 12, again to help ensure downward pressure is concentrated on the tips of the microneedles 16 during deployment. The skin S may deform to quite a variable extent (for example depending on anatomical location, skin type, user pressure, subdermal fat level, etc.) during this action and thus it is important that sufficient pressure is transferred to the microneedles 16 to achieve insertion as the patch 12 is displaced from the undeployed to the deployed state. Again it will be understood that the dimensions and geometry of the applicator 14 may be significantly varied while still retaining the functionality described herein. For example the applicator 14 could be designed with a so called "pistol grip" form to be gripped in the palm and having two parts that may be squeezed together by the user to effect the above described displacement. Such a configuration would for example be suited to users having reduced manual dexterity. As with the patch 12, the body portions 22, 24 may be formed from any suitable material or combination of materials, and are preferably formed from moulded plastic. The body portions 22, 24 may also include instructional iconography and/or colour coding or the like in order to assist the user, for example to effect deployment of the patch 12 and as will be described hereinafter.

Referring in particular to Figures 2, 7 and 8 the underside or skin facing side of the applicator 14 is shown, and against which, in use, the patch 12 is retained. The applicator 14 comprises an actuator partly defined by a pair of tabs 28, 30, one of which is fixed to or formed integrally with the first body portion 22 and the other tab 30 being fixed to or formed integrally with the second body portion 24. A channel 32 is provided in the underside of the first body portion 22 to accommodate displacement of the second tab 30 as the first and second body portions 22, 24 undergo relative longitudinal displacement. In this way relative displacement of the first and second body portions 22, 24 results in relative displacement of the first and second tabs 28, 30. Each tab 28, 30 defines a first surface 34, the pair of first surfaces 34 being substantially parallel to one another, the first surfaces 34 facing away from one another or facing outwardly. The first surfaces 34 are also oriented obliquely of the longitudinal axis LL of the system 10, and thus obliquely of the direction of relative displacement between the first and second body portions 22, 24 and therefore between the first and second tabs 28, 30. In the embodiment illustrated the tabs 28, 30 themselves are provided in an oblique orientation relative to the longitudinal axis LL, but this is not essential once the first surfaces 34 are obliquely oriented. In other words, the tabs 28, 30 may themselves be orientated parallel to the longitudinal axis LL while the first surfaces 34 extend obliquely, resulting in each of the tabs 28, 30 having a wedge shape increasing in thickness from one end to the other in the longitudinal direction.

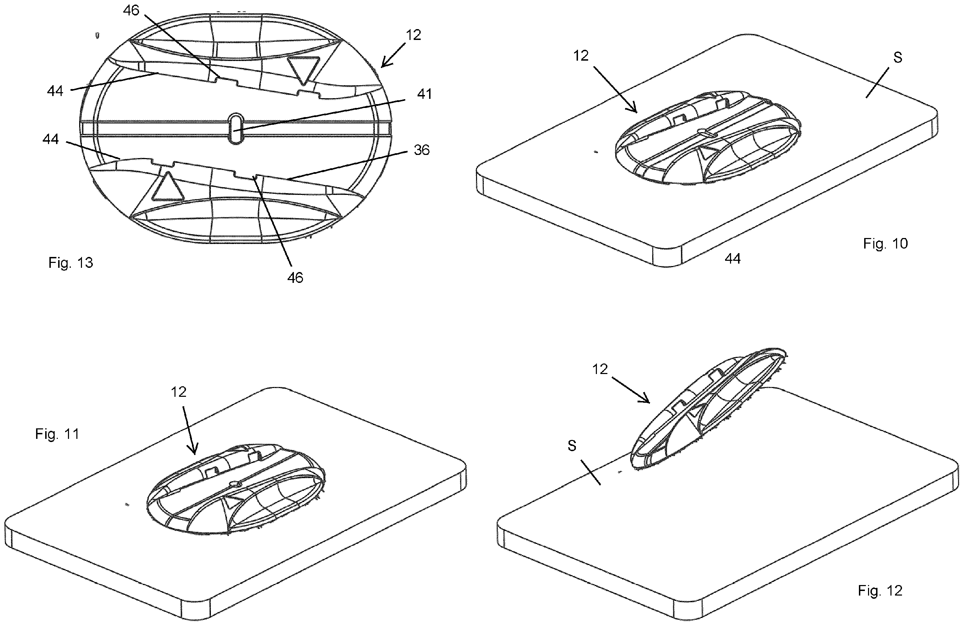

The actuator is further defined by a corresponding pair of substantially parallel second surfaces 36 one of which is defined on the upper face of each patch half 18, 20, as most clearly illustrated in Figures 10, 11, 12 and 13. Each half 18, 20 of the patch 12 comprises an upstanding protrusion 38 whose outer edge defines the respective second surface 36, and which is oriented to extend obliquely to the longitudinal axis LL, and at substantially the same angle as the first surfaces. In this way when the patch 12 is secured to the underside of the applicator 14 as described hereinafter, the first surfaces 34 are captured between and are in face to face engagement with the second surfaces 36 as visible in Figure 5. It will therefore be appreciated that in displacing the first surfaces in the longitudinal direction by displacing or compressing the body portions 22, 24, the first surfaces 34 will slide past and bear against the second surfaces 36 affecting a cam action, forcing the second surfaces away from one another in a direction transverse to the longitudinal axis LL, thus resulting in displacement of the patch halves 18, 20 from the closed to the open position and so the microneedles will be displaced from the undeployed to the deployed state. Provided that this action is undertaken with sufficient downward pressure on the skin S the microneedles 16 will penetrate and become at least partially embedded in the skin S.

The application system 10 is therefore preferably provided with a releasable lock in the form of a spring biased pin 40 extending from the applicator 14, preferably in-between the tabs 28, 30, to contact the upper face of the patch 12. The pin 40 is arranged to prevent relative displacement of the body portions 22, 24 from the extended to retracted positions until a threshold pressure is applied to the pin 40 to overcome the spring bias, allowing the pin 40 to depressed, which releases the lock and therefore permits the relative displacement of the body portions 22, 24. The threshold pressure is thus chosen to be at or slightly above the pressure required to be applied at the tip of the microneedles 16 to ensure tissue insertion. The tip of the pin 40 is seated in a well 41 provided in the upper face of the patch 12, and the spring bias of the pin against the patch 12 during deployment thus acts to prevent relative translation of the applicator 14 and patch 12. It will be appreciated that any other suitable arrangement of lock may be utilised to provide the above described functionality.

In order to retain the patch 12 on the applicator prior to deployment of the patch, the system 10 comprises a coupling in the form of a pair of keys 42 provided on each tab 28, 30 and a corresponding pair of keyways 44 provided on the patch 12, one on each patch half 18, 20, within and along which the respective pair of keys 42 is slidably and releasably captured. In the embodiment illustrated the coupling and actuator are integrated with one another, whereby the keyways 44 are formed beneath an overhang on the protrusion 38 which defines the respective second surface 36 and the keys are provided on the tabs 28, 30, effectively forming a stepped extension of the respective first surface 34. The keyway 44 therefor also extends in the same oblique orientation relative to the longitudinal axis LL, and the same cam action will occur as the keys 42 travel along and bear against the keyway 44. The oblique angle can be selected to provide a mechanical advantage in translating the relatively large displacement of the first and second body portions 22, 24 of the applicator 14 into relative displacement of the patch halves 18, 20. The microneedles are only required to be displaced a relatively short distance, for example one fifth the stroke or displacement of the body portions 22, 24, but with sufficient force to pierce the skin S, and so this oblique angle of the first and second surfaces 34, 26 reduces the distance of displacement but increases the force applied to the microneedles 16.

A pair of openings 46 are located in an upper wall of each keyway 44 which are shaped to permit the keys 42 to pass therethrough, and are located along the keyway 44 such as to be in alignment with the keys 42 only when the tabs 28, 30 have been displaced into the position as illustrated in Figure 8, that is when the body portions 22, 24 have been compressed together by the user. Prior to this, with the body portions 22, 24 extended relative to one another as in Figure 7, the openings 46 are offset relative to the keys 42 and thus the keys 42 are captured in the keyways 44, thereby acting to retain the patch 12 on the underside of the applicator 14. When the user presses the patch 12 against the skin S with sufficient pressure to overcome the locking pin 40, the user can then displace the body portions 22, 24 together through a pinching action between thumb and index finger, which action results in the relative displacement of the tabs 28, 30. This affects the above described cam action forcing the patch halves 18, 20 apart to drive the microneedles 16 into the skin. The corresponding movement of the keys 42 along the keyways 44 during this action simultaneously brings the keys 42 into register with the openings 46, allowing the applicator 14 to be separated from the deployed patch 12.

In order to prevent a user from consciously or inadvertently displacing the body portions 22, 24 back towards the extended position, particularly while still engaged with the patch 12, the applicator 14 is preferably provided with at least one, and preferably a pair of non return locks 48 comprising a stepped track 50 formed in the lower face of the first body portion 22 and a resiliently deformable follower 52 projecting into the track 50 from the lower face of the second body portion 24. As the first and second body portions 22, 24 are compressed into the retracted position shown in Figure 8 the follower 52 will move along the track 50 and be irreversibly displaced into a notch 54 at the end of the track 50, preventing the follower 52 from moving in the opposite direction back along the track 50 and therefore preventing the body portions 22, 24 from being displaced back towards the extended state shown in Figure 7. However it should also be understood that the non return locks 48 are optional features and in various embodiments may be omitted as the system may be designed to be reloaded with another patch and reused.

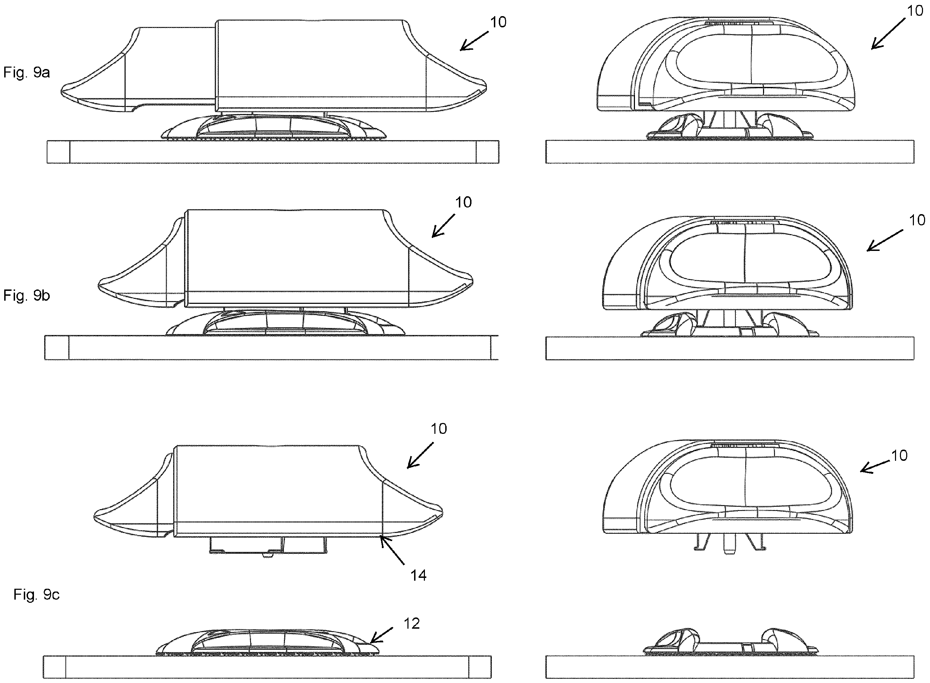

Referring to Figure 9 the sequence of steps for applying the patch 12 are illustrated in side and end elevations. Before the first step a user will unpack the system 10, which may include removal of some form of protective covering overlying the microneedles 16, and will then locate the patch 12 on an appropriate target section of skin S as shown in Figure 9a. With sufficient pressure applied to the skin S by the patch 12 the pin 40 lock will be released and the user can pinch together the body portions 22, 24 as shown in Figure 9b. This will result in the microneedles 16 being embedded into the skin S, and will align the keys 42 with the openings 46, allowing the applicator 14 to be removed as shown in Figure 9c. The patch 12 will therefore remain in place on the skin S to perform the requisite function, whether drug delivery or otherwise.

When it is time to remove the patch 12, and referring to Figures 10 to 13, the user can simply pinch the patch halves 18, 20 together as indicated by the arrow iconography on the upper face of the patch 12. The patch 12 is preferably provided with a depression 56 in the upper face of each half 18, 20 which receive the users fingertips during the removal procedure to provide increased purchase on the otherwise relatively low profile patch 12. The force of removal is preferably predominantly governed by a latching mechanism (not shown) provided in the patch 12 and acting to prevent the patch halves 18, 20 from being closed until a threshold pressure is applied. That is, the microneedles 16 can be removed from the skin S with minimal removal force relative to the insertion force. The contour of the depressions 56 are such that the user inadvertently presses down on the skin S which increases the grip obtained. This normal force may be used to help overcome the latching mechanism (not shown). The patch 12 can then be removed and discarded.

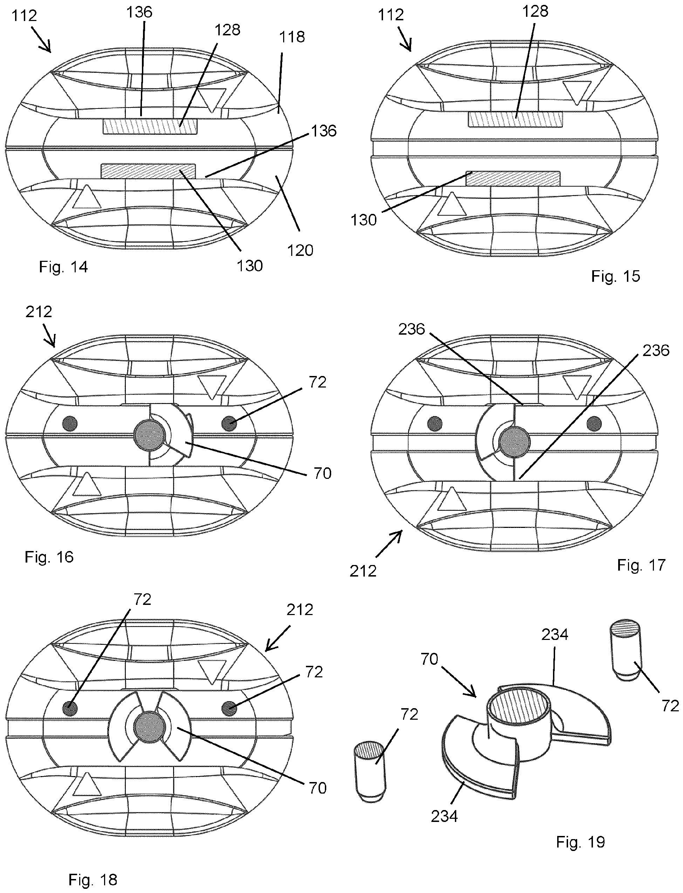

Referring now to Figures 14 and 15 there is illustrated a microneedle patch according to an alternative embodiment of the invention, generally indicated as 112. In this alternative embodiment like components have been accorded like reference numerals and unless otherwise stated perform a like function. Figures 14 and 15 show only the upper surface of the patch 112, the underside and microneedles (not shown) carried by the patch being the same as described with reference to the patch 12.

The patch 112 comprises slidably engaged patch halves 118,120 and include parallel second surfaces 136 which are also parallel, in use, with a longitudinal axis of the applicator (not shown). The applicator must therefore be adapted to displace a pair of first surfaces (not shown) of the applicator against the second surfaces 136 such as to displace the patch halves 118, 120 from the undeployed state shown in Figure 14 into the deployed stage as shown in Figure 15, and to then release the patch 112 to allow removal of the applicator. First and second tabs 128, 130 of the applicator (not shown) and which define the first surfaces are illustrates in hatched detail. Various mechanical configurations may be employed to achieve this functionality, and may for example include a third actuation state in which the first surfaces (not shown) of the actuator are drawn or otherwise displaced back towards one another such as to disengage the keys (not shown) from the keyways (not shown) on the patch 112.

The patch 112 may be removed from the skin following use in the same manner as described above for the patch 12.

Referring now to Figures 16 to 19 there is illustrated a microneedle patch according to a further alternative embodiment of the invention, generally indicated as 212. In this alternative embodiment like components have been accorded like reference numerals and unless otherwise stated perform a like function. Figures 16 to 18 show only the upper surface of the patch 212, the underside and microneedles (not shown) carried by the patch being the same as described with reference to the patch 12.

The patch 212 comprises slidably engaged patch halves 218, 220 and includes parallel second surfaces 236 which are also parallel, in use, with a longitudinal axis of the applicator (not shown). The system thus includes an actuator in the form of a cam 70 provided as part of the patch 12 but which may be coupled with the applicator to apply a rotational translation thereto during deployment. The cam 70 defines a pair of first surfaces 234 engageable against the second surfaces 236 in order to open the patch 212. The cam 70 is shown in isolation in Figure 19, along with a pair of pins 72 which extend in use from the applicator (not shown) to releasably engage the patch 212. The pins 72 provide rotational constraint and ensure that the applicator does not rotate relative to the patch 212 during deployment, which would otherwise adversely affect the coupled lateral motion that is required for successful microneedle deployment.

The cam 70 may be rotated by converting linear displacement of the applicator (not shown) as described above into rotary displacement through any suitable conventional mechanical means (not shown). Rotation of the cam 70 will thus displace the patch 212 from the undeployed state shown in Figure 16 into the deployed stage as shown in Figure 17, and to then release the patch 112 to allow removal of the applicator.

To achieve removal of the patch 212 following use a third configuration is required, in which the cam 70 is arranged such that the first surfaces 234 are disengaged from the second surfaces 236, allowing the patch 212 to be closed for removal from the skin. The patch 212 may then be removed in the same manner as described above for the patch 12.

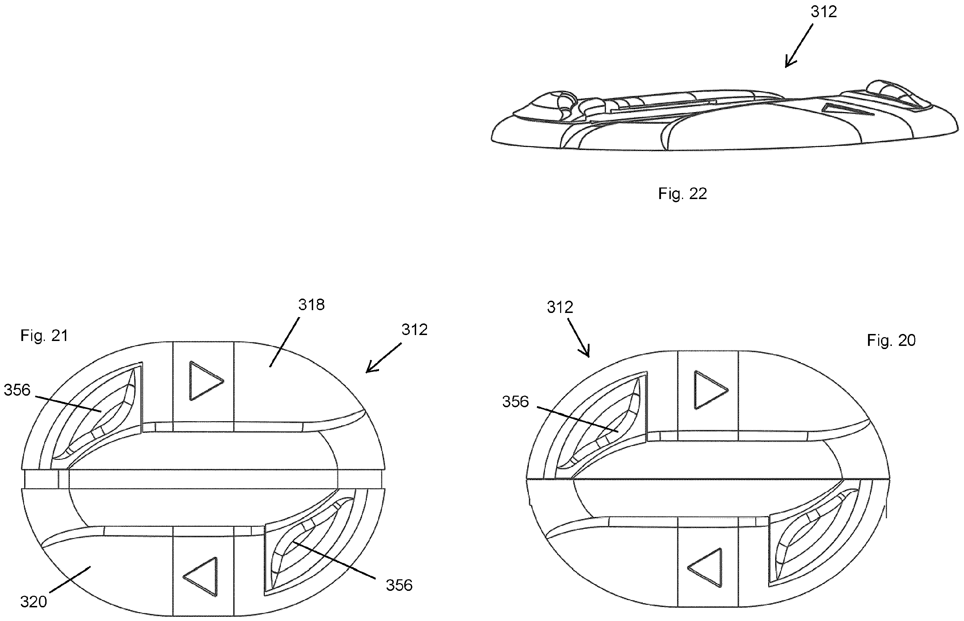

In a further embodiment illustrated in Figures 20 to 23 a patch 312 is provided which exhibits features that facilitate an out-of-plane squeeze for removal of the patch 312 from the skin. The patch 312 comprises patch halves 318, 320 each of which defines a contoured tab 356 to allow the patch 312 to be gripped between the finger and thumb of the use and squeezed for removal from the skin. In this embodiment this action may be spring assisted. That is, a spring is under tension when the patch 312 is deployed and this potential energy released during removal when the tabs 356 are squeezed.

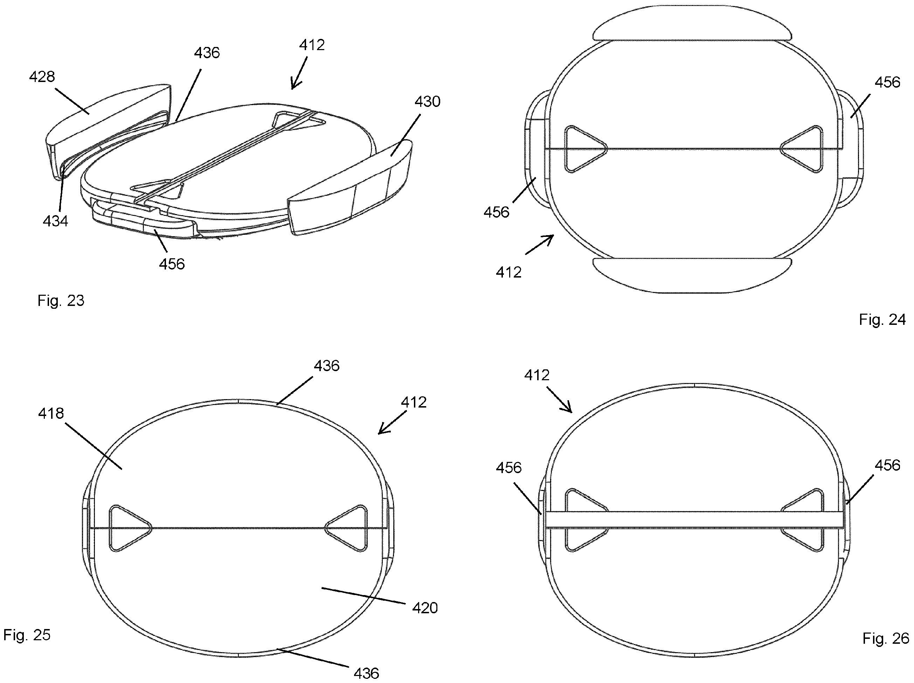

Referring now to Figures 23 to 26 there is illustrated an additional embodiment of a microneedle patch, generally indicated as 412. In this alternative embodiment like components have been accorded like reference numerals and unless otherwise stated perform a like function. Again Figures 23 to 26 show only the upper surface of the patch 412, the underside and microneedles (not shown) carried by the patch 412 being the same as described with reference to the patch 12.

The patch 412 comprises a pair of patch halves 418, 420 which are displaceable relative to one another between an undeployed state as illustrated in Figures 23 and 26 in which the patch halves 418, 420 are partially separated or displaced outwardly of one another, and a deployed state as illustrated in Figures 24 and 25 in which the halves 418, 420 are pressed together or closed. However in this embodiment the patch 412 is adapted such that an applicator, which is partially illustrates in Figures 23 and 24 in the form of a pair of tabs 428, 430, is arranged to engage opposed exterior edges or surfaces of the patch 412. Thus the tabs 428, 430 defines a pair of first surfaces 434, while the outer edge of the patch 412 defines a cooperating pair of second surfaces 436 which may be engaged and displaced by the first surfaces 434 in order to displace the patch 412 from the undeployed to the deployed state. In this embodiment the patch 412 is thus gripped between the tabs 428, 430 in order to retain the patch 412 in the applicator in the undeployed state, and is then effectively squeezed between the tabs 428, 430 as they are displaced towards one another by any suitable mechanical action of the applicator. This squeezing action, while the patch 412 is being pressed against a tissue substrate, causes the patch 412 to close and thus be deployed onto the tissue substrate as hereinbefore described.

In order to assist in removing the patch 412 from a tissue substrate such as the skin the patch 412 preferably comprises an opposed pair of protrusions 456 which may be displaced towards one another to affect a mechanical action of separating or forcing open the patch halves 418, 420, as illustrates in Figure 26.

It will therefore be understood that the patch 412 is configured to be deployed using a 'closing' action and removed using an 'opening' action. In this scenario the first surfaces 434 are most preferably and practically located laterally outboard of the second surfaces 436 on the patch 412. This removal opening action could be spring-assisted in a further embodiment.

It is further envisaged that the system of the invention may be modified such that at the moment of deployment of the patch a mass is simultaneously released perpendicular to the skin S. This energy release momentarily increases downward pressure and may increase initial penetration of the microneedles.

## Claims

1. A microneedle patch application system comprising a microneedle patch for deployment on a tissue substrate and an applicator operable to deploy the microneedle patch onto said substrate; wherein the microneedle patch comprises two sets of microneedle arrays with the microneedles in one array pointed in generally the opposite direction to the microneedles in the other array, the arrays being displaceable relative to one another between a undeployed state and a deployed state; a coupling operable to releasably retain the microneedle patch on the applicator; and an actuator operable to affect displacement of the microneedle arrays from the undeployed to the deployed state when the patch is retained on the applicator.

2. A microneedle patch application system according to claim 1 in which the applicator is deformable and said deformation affects operation of the actuator and/or the coupling.

3. A microneedle patch application system according to claim 2 in which the applicator comprises a first portion and a second portion and deformation of the applicator is affected by relative displacement between the first and second portions.

4. A microneedle patch application system according to claim 3 comprising a lock operable to prevent relative displacement of the first and second portions until a threshold pressure is applied to the applicator from the patch.

5. A microneedle patch application system according to claim 3 or 4 comprising a non return lock operable to prevent the first and second portions from undergoing relative displacement once the arrays have been displaced into the deployed state.

6. A microneedle patch application system according to any of claims 2 to 5 in which the applicator is manually deformable.

7. A microneedle patch application system according to any preceding claim in which the applicator is operable such that actuation of the actuator affects actuation of the coupling.

8. A microneedle patch application system according to any preceding claim in which the coupling and the actuator are integrated with one another such as to facilitate unitary operation thereof.

9. A microneedle patch application system according to any preceding claim in which the actuator comprises a pair of first surfaces on the applicator which are displaceable relative to one another, and a pair of second surfaces each of which is secured to one of the arrays, the second surfaces being engageable with and displaceable by the first surfaces.

10. A microneedle patch application system according to claim 9 in which the pair of first surfaces are displaceable relative to one another by means of a linear translation and/or a rotational translation of the pair of first surfaces.

11. A microneedle patch application system according to claim 9 or 10 in which the pair of first surfaces are substantially parallel and displaceable relative to one another in a direction oblique to the first surfaces, the pair of second surfaces are substantially parallel and each of which is secured to one of the arrays, the second surfaces being displaceable relative to one another in a direction oblique to the second surfaces, the first and second surfaces being in face to face engagement when the patch is retained on the applicator.

12. A microneedle patch application system according to any preceding claim in which the coupling comprises at least one key on the applicator or patch and a corresponding keyway on the other of the applicator or patch within and along which the at least one key is slidably and releasably captured.

13. A microneedle patch application system according to claim 12 when dependent on claim 11 in which the keyway is at least partially defined by one of the second surfaces.

14. A microneedle patch application system according to claim 12 or 13 when dependent on claim 11 in which the key is releasable from the keyway following a predetermined relative displacement of the first surfaces relative to the second surfaces.

15. A method of applying a microneedle patch to a tissue substrate comprising the steps of releasably securing a microneedle patch to an applicator, the patch comprising two sets of microneedle arrays with the microneedles in one array pointed in generally the opposite direction to the microneedles in the other array; pressing the arrays of microneedles against the tissue substrate; utilising the applicator to displace the pair of arrays relative to one another between a undeployed state and an deployed state such as to at least partially embed the microneedles in the tissue substrate; and releasing the actuator from the patch.
